# EUROPEAN PATENT SPECIFICATION

(11) **EP 0 669 834 B1**
(45) Date of publication and mention of the grant of the patent: **08.09.1999**
(21) Application number: 94900578.9
(22) Date of filing: 09.11.1993
(51) Int. Cl.: A61K 38/45

(54) **USE OF TOPICALLY APPLIED FACTOR XIII FOR INHIBITING HEMORRHAGE**
VERWENDUNG VON LOKAL APPLIZIERTEM FAKTOR XIII ZUR VERHINDERUNG VON BLUTUNGEN
PROCEDE ET UTILISATION DU FACTEUR XIII EN APPLICATION LOCALE POUR EMPECHER UNE HEMORRAGIE

(30) Priority: 12.11.1992 US 975026
(43) Date of publication of application: 06.09.1995
(73) Proprietor: ZymoGenetics, Inc., Seattle, WA 98102 (US); MAYBERG, Marc R., Seattle, WA 98112 (US)
(72) Inventor: MAYBERG, Marc, R., Seattle, WA 98112 (US); EDWARDS, Martin, William, Belle Mead, NJ 08502 (US)
(74) Representative: Noergaard, Torsten
(86) International application number: US9310758
(87) International publication number: WO9411022

(56) References cited:
- EP-A- 0 330 049
- WO-A-89/01512
- DE-A- 2 914 822
- DE-A- 3 829 524
- NEUROSURGERY vol. 32, no. 4 , April 1993 pages 630 - 634 V. LAOHAPRASIT ET AL. 'Prevention of postoperative intracerebral hemorrhage with topical recombinant factor XIII in the rat'
- NO SHINKEI GEKA, APR 1985, VOL. 13, NO. 4, PAGE(S) 367-73, FUKUMOTO T ET AL '[The use of fibrin glue in neurosurgical operations]'
- DTSCH ZAHNARZTL Z, SEP 1977, VOL. 32, NO. 9, PAGE(S) 676-9, KIRSCHNER H ET AL 'Heilung von Zahnextraktionswunden bei lokaler und systemischer Anwendung von Plasmafaktor XIII im Tierexperiment.'
- THROMB DIATH HAEMORRH, DEC 31 1974, VOL. 32, NO. 2-3, PAGE(S) 578-81, MARKTL W ET AL 'The effect of factor XIII on wound granulation in the rat.'
- WIEN KLIN WOCHENSCHR SUPPL, 197, VOL. 49, PAGE(S) 3-18, SPANGLER HP 'Tissue adhesion and local haemostasis using fibrinogen, thrombin and clotting factor xiii. (experimental investigations--clinical experience) (author's transl)]'
- NEUROCHIRURGICA vol. 34, no. 4 , 1991 pages 107 - 110 A. THIE ET AL 'Factor XIII concentrate for prevention of recurrent subarachnoid hemorrhage: results of a multicenter pilot study'
- CHEMICAL ABSTRACTS, vol. 102, no. 14, 8 April 1985, Columbus, Ohio, US; abstract no. 119565c, SUGITACHI A. ET AL. 'New materials enhancing local accumulation of fibrin. Topically static, oncolytic agent' page 395 ; & KETSUEKI TO MYAKKAN vol. 15, no. 5 , 1984 pages 559 - 561
- BIOCHEMISTRY vol. 29 , 1990 pages 1861 - 1869 P.D. BISHOP ET AL. 'Expression, purification and characterization of Human Factor XIII in Saccharomyces cerevisiae'

## Description

### Background of the Invention

Hemorrhage (blood loss) is a serious complication of major surgery. In neurosurgical patients, for example, post-operative intracerebral hemorrhage results in severe pressure on the brain that can rapidly lead to neurological damage, loss of consciousness and death. Patients who have coagulopathy, such as those suffering from disseminated intravascular coagulation or congenital clotting defects, or those undergoing concurrent anticoagulation therapy, are at higher risk for spontaneous and post-operative intracranial hemorrhage (Eyster et al., Blood 51: 1179-1188, 1978; Goodnight et al., N. Eng. J. Med. 290: 1043-1047, 1974; Kaufman et al., Neurosurg. 7: 445-450, 1980).

Depending on the site and nature of the neurosurgical procedure, postoperative bleeding may be intraventricular, intraparenchymal, subdural, or extradural in location. The existence of a cavity, whether it be an excavated tumor bed; one of the ventricles; or a potential space, such as the subdural, epidural or subarachnoid spaces, represents a vulnerable area for significant clot formation. Abetted by a rise in systemic blood pressure and loss of local autoregulation, small arterioles at the site of brain surgery may dilate in a delayed fashion after temporary spasm due to operative manipulation. Venous surface bleeding in brain is usually controlled by meticulous bipolar electrocoagulation and selective use of coagulation activators such as hemostatic sponges soaked in thrombin, oxidized cellulose, microfibrillary collagen, or muscle stamps. However, hemostatic sponges may seal the tumor bed and allow bleeding into the underlying brain, as reported by Mullan (JAMA 182: 105-112, 1962). Despite these surgical adjuncts, post-operative hemorrhage remains a significant complication in neurosurgical patients with normal clotting parameters, with an incidence estimated at 10 to 30% (Yazargil, Microneurosurgery IIIB: 169-203, 358-366, 1988). The risk of delayed hemorrhage is further potentiated after surgery in patients with abnormal coagulation parameters, including hemophilia, disseminated intravascular coagulation (Goodnight et al., ibid.; Kaufman et al., ibid.) and therapeutic anticoagulation.

Various wound healing compositions based on combinations of coagulation factors and/or other hemostatic agents have been disclosed. U.S. Patent No. 4,453,939 discloses a composition for sealing and healing wounds comprising factor XIII, fibrinogen, thrombin and collagen. U.S. Patent No. 4,427,650 discloses a powdered tissue adhesive comprising fibrinogen and thrombin and/or prothrombin. PCT Publication WO 90/13320 discloses a hemostatic sponge containing a hemostatically effective amount of thrombin, which may further contain factor XIIIa and other coagulation factors. These products, however, are derived from human and/or animal blood plasma, and consequently present risks of viral contamination and antigenicity.

A certain percentage of post-operative neurosurgical hemorrhages are presumed to occur when an existing thrombus at the corticectomy site is removed by endogenous plasmin-mediated thrombolysis. A novel potential therapy for cerebral vasospasm, intracisternal administration of recombinant tissue plasminogen activator (Findlay et al., Can. J. Neurol. Sci. 16: 28-40, 1989; Findlay et al., J. Neurosurg. 69: 723-735, 1988), might also increase the risk of hemorrhage due to augmented thrombolysis at sites of cortical injury.

There remains a need in the art for therapeutic methods that reduce the incidence of post-operative hemorrhage, especially in high-risk settings. The present invention provides such methods through the use of topically applied factor XIII. The invention further provides compositions useful in reducing post-operative hemorrhage, as well as methods and compositions for reducing hemorrhage in other types of wounds.

### Summary of the Invention

In its broadest aspect, the present invention is directed to methods for inhibiting hemorrhage through the use of topically applied factor XIII.

Within one aspect, the present invention provides methods for inhibiting delayed bleeding of a wound. The methods comprise topically applying to the wound an effective amount of a composition comprising factor XIII in a biologically compatible vehicle, wherein the composition is substantially free of other blood coagulation factors and hemostatic agents.

Within a related aspect, the invention provides methods for inhibiting post-operative hemorrhage comprising (a) treating a surgical site in a patient to induce hemostasis, and (b) topically applying to the treated surgical site an effective amount of factor XIII in a biologically compatible vehicle. Within one embodiment, the patient is coagulopathic due to, for example, administration of an exogenous anticoagulant or fibrinolytic compound. Within another embodiment, the surgical site is intracranial. Within another embodiment, the factor XIII is recombinant factor XIII. Within a related embodiment, the factor XIII is factor XIII **a**_{**2**} dimer. Within additional embodiments, the biologically compatible vehicle is an aqueous diluent or a gel.

Within another aspect, the present invention provides methods for inhibiting post-operative hemorrhage, comprising topically applying to a surgical site in a patient a composition consisting essentially of factor XIII in a biologically compatible vehicle.

These and other aspects of the invention will become evident upon reference to the following detailed description.

### Detailed Description of the Invention

Prior to setting forth the invention, it may be helpful to an understanding thereof to set forth definitions of certain terms to be used hereinafter.

Blood coagulation factors: Procoagulant proteins or precursors thereof, which participate in the blood coagulation cascade. Blood coagulation factors include fibrinogen, thrombin, thromboplastin, factor V, factor VII, factor VIII, factor IX, factor X, factor XI, factor XII and factor XIII.

Coagulopathic: Having an increased risk of bleeding due to, for example, a congenital deficiency, disease, or therapeutic treatment.

Hemostasis: The cessation of hemorrhage due to the formation of a blood clot.

Topical: Local, as contrasted to systemic. A topical medicament is one applied directly to the site where it is needed, rather than applied through the circulatory system.

Intracranial hemorrhage: Extravasation of blood into one or more of several potential spaces within the cranial cavity. These potential spaces include (a) epidural, located between the dura and the skull; (b) subdural, between the arachnoid and the dura; (c) subarachnoid, between the brain surface and the arachnoid; (d) intraparenchymal, within the substance of the brain; and (e) intraventricular, within the ventricles of the brain.

The present invention provides improved methods and compositions for reducing hemorrhage from wounds, including surgical wounds. These methods and compositions are particularly useful in reducing delayed bleeding, that is the resumption of bleeding within a wound in which hemostasis has apparently been induced. Of particular interest is the reduction of post-operative hemorrhage in patients. The methods and compositions of the present invention are particularly useful in reducing or preventing intracranial hemorrhage in patients who have undergone neurosurgery. The methods are useful in patients having defects in blood coagulation, including those suffering from disseminated intravascular coagulation (DIC) or congenital clotting defects, and in those receiving concurrent anticoagulant or antifibrinolytic therapy.

The inventors have found that topical application of factor XIII can be used to reduce the risk of post-operative hemorrhage, especially post-operative intracranial hemorrhage in high-risk patients with coagulopathy. It has been found that factor XIII is effective when applied as a composition that is substantially free of other blood coagulation factors and hemostatic agents, that is, free of hemostatically effective amounts of such other factors and agents.

To determine the efficacy of topically applied factor XIII in preventing post-operative hemorrhage, a rat model for experimental craniotomy and standardized bilateral frontal corticectomy was developed. In 25 rats, recombinant factor XIII or placebo solution were topically applied to corticectomy cavities after hemostasis was achieved. In 20 rats, intraperitoneal heparin sulfate (100 units/kg/hour) was initiated 3 days after surgery and continually administered by Alzet pump for 7 days, compared to a control group of 5 rats receiving intraperitoneal saline. The volume of intracranial hemorrhage was quantitatively determined from coronal sections using automated image analysis. Large (>50 mm³) intracerebral hemorrhages were significantly more frequent in placebo (60%) compared to recombinant factor XIII (15%) treated lesions (p<0.01).

Factor XIII (also known as "fibrinoligase" [Lorand et al., Prog. Hemost. Thromb. 5: 245-290, 1980] and "fibrin stabilizing factor [Curtis and Lorand, Methods Enzymol. 45: 177-191, 1976]) is characterized by its ability, when activated, to form intermolecular γ-glutamyl-ε-lysine cross links between side chains of fibrin molecules and between other substrates. The enzyme exists in plasma as a tetrameric zymogen of two **a** subunits and two **b** subunits (designated **a**₂**b**₂), but is found in other tissue as an **a**₂ dimer. Either of these zymogen forms, or activated factor XIII (factor XIIIa), may be used within the present invention, as well as genetically engineered variants of factor XIII that retain its characteristic cross-linking activity.

Factor XIII for use within the present invention may be prepared from plasma according to known methods, such as those disclosed by Cooke and Holbrook (Biochem. J. 141: 79-84, 1974) and Curtis and Lorand (Methods Enzymol. 45: 177-191, 1976),. The **a**₂ dimer form of factor XIII may be prepared from placenta as disclosed in U.S. Patents 3,904,751; 3,931,399; 4,597,899 and 4,285,933. It is preferred, however, to use recombinant factor XIII so as to avoid to the use of blood- or tissue-derived products that carry a risk of disease transmission.

Methods for preparing recombinant factor XIII are known in the art. See, for example, Davie et al., EP 268,772 and Grundmann et al., AU-A-69896/87, in their entirety. Recombinant factor XIII **a**₂ dimer may be prepared cytoplasmically in the yeast Saccharomyces cerevisiae as disclosed in copending United States Patent Application Serial No. 07/741,263.

The transformed cells are cultured, harvested and lysed, and a cleared lysate is prepared. The lysate is fractionated by anion exchange chromatography at neutral to slightly alkaline pH using a column of derivatized agarose, such as DEAE Fast-Flow Sepharose™ (Pharmacia) or the like. Factor XIII is then precipitated from the column eluate by concentrating the eluate and adjusting the pH to 5.2-5.5, such as by diafiltration against ammonium succinate buffer. The precipitate is then dissolved and further purified using conventional chromatographic techniques, such as gel filtration and hydrophobic interaction chromatography.

As will be appreciated by those skilled in the art, it is preferred to use a factor XIII protein syngeneic with the patient in order to reduce the risk of inducing an immune response. Preparation and characterization of non-human factor XIII has been disclosed by Nakamura et al. (J. Biochem. 78: 1247-1266, 1975). The present invention encompasses the use of such factor XIII proteins within veterinary procedures.

Within the methods of the present invention, an effective amount of factor XIII is combined with a biologically compatible vehicle and topically applied to a wound or surgical site. An "effective amount" of factor XIII is that amount sufficient to reduce the incidence of medically significant hemorrhages. As will be appreciated by those skilled in the art, the size of a hemorrhage that is medically significant will vary according to location. For example, even a very small post-operative intracranial hemorrhage in the brain can produce significant deleterious effects, damaging the surrounding brain tissue by direct pressure. Thus, any reduction in the incidence or volume of intracranial hemorrhage would be beneficial. In the chest or abdominal cavity, much larger volumes of hemorrhage can be tolerated because the primary risk factor is loss of blood. In general, factor XIII is applied at a concentration in excess of normal blood levels (10 µg/ml), preferably between 0.1 mg/ml and 100 mg/ml, more preferably about 1-10 mg/ml. The factor XIII composition is preferably applied in an amount at least sufficient to cover the wound surface or fill the wound cavity. Suitable vehicles include sterile, non-pyrogenic aqueous diluents, such as sterile water for injection, sterile buffered solutions and sterile saline. For use in surgical fields where considerable amounts of fluid are present and simple aqueous solutions might be diluted or washed away, it is preferred to use a more viscous carrier. Suitable carriers in this regard include gels, pastes and ointments ordinarily used for topical delivery of pharmaceutical compounds. Preferred carriers include solutions of water soluble polymeric materials, particularly cellulose-based gels, such as methyl cellulose or hydroxyethyl cellulose gels.

The factor XIII compositions described above are administered to the patient prior to closing the surgical opening, preferably after the surgical site is treated to induce hemostasis. Hemostasis is induced by conventional surgical techniques, such as application of a hemostatic sponge (e.g. Gelfoam^{®}, The Upjohn Co., Kalamazoo, MI; porous hydrogel sponges, U.S. Patent No. 4,002,173), microfibrillar collagen (e.g. Avitene^{®}, Alcon Laboratories, Inc., Fort Worth, TX), collagen fleece, oxidized cellulose (e.g. Surgicel™, Johnson & Johnson, New Brunswick, NJ) or the like. Hemostasis may also be induced by application of topical coagulation factors (e.g. thrombin), cauterization, electrocoagulation, or other known procedures. The factor XIII composition (e.g. an aqueous solution or a gel) is then applied to the surgical site, and the wound is closed, reducing the potential rebleeding space by mechanical apposition if possible.

The methods of the present invention are particularly useful at surgical sites and in patients that present a high risk of rebleeding. High-risk sites include tissues or organs with a rich vascular supply, in which post-operative bleeding is a common complication. Specific examples include the central nervous system, liver, heart and lung. High-risk patients include those undergoing anticoagulant or antifibrinolytic therapy and those with coagulopathic conditions such as disseminated intravascular coagulation or hemophilia.

The invention is further illustrated by the following non-limiting example.

### EXAMPLE

Twenty-five male Sprague-Dawley rats weighing 450-500 gm were used for the study. All procedures were performed using protocols approved by the Animal Care Committee of the Seattle Veterans Administration Medical Center. Rats were anesthetized (50 mg/kg pentobarbital, i.p.), allowed to breathe spontaneously, and a standardized bilateral frontal craniectomy was performed through a midline cranial incision. Both frontal lobes were exposed, and bilateral standardized frontal lesions measuring 24 cubic millimeters (2x3x4 mm) were made using a calibrated scalpel and cup forceps. The pial surface of the corticectomy wound was coagulated with bipolar electocautery, the cavity was lined with Surgicel™ (Johnson & Johnson Products Inc., New Brunswick, NJ), and complete hemostasis was verified by observation for 15 minutes. Twenty five µl of recombinant factor XIII **a**₂ dimer (50 µg in 30% glycerol and phosphate buffered saline) or placebo vehicle were topically applied to either corticectomy cavity in a blinded, randomized fashion. The dural opening was covered by Gelfoam® (Upjohn Co., Kalamazoo, MI), and the wound was closed with sutures. After surgery each rat was housed individually in a room with controlled temperature (72°F) and light exposure (12 hours of light each day); food and water were available *ad libitum.*

On the third post-operative day, rats were anesthetized by methoxyflurane inhalation, and an Alzet pump (model 2001; Alza Corporation, Palo Alto, CA) containing heparin solution (N=20; 227µl; 40,000 units/ml) or normal saline (N=5; 227 µl) was placed into the abdominal cavity of each rat, and the incisions were closed with sutures. The Alzet osmotic pumps were calibrated to provide continuous outflow at 1.01±0.03 µl/hr, in this case providing intraperitoneal heparin administration at rates of 100 units/kg/hr to produce consistent elevations in venous activated partial thromboplastin time (aPTT) and a significant incidence of intracerebral hemorrhage. Venous blood was obtained from tail veins immediately prior to Alzet implantation for baseline aPTT and 3 days thereafter. A third blood sample for aPTT was obtained by cardiac puncture at the time of euthanasia.

The mean aPTT of control rats in this experiment was 19.3±3.6 seconds. Intraperitoneal heparin at 100 units/kg/hour maintained a consistent level of anticoagulation (mean aPTT=124.6±21.2 seconds).

After 7 days of continuous intraperitoneal heparin or saline administration, rats were anesthetized as above and the brains perfused by intracardiac infusion of 0.03 M phosphate buffered Ringer's solution, followed by 200 ml of 4% paraformaldehyde in phosphate buffer (pH=7.4) at mean physiologic arterial pressure via a left ventricular cardiac puncture. After fixation in 10% formalin, brains were embedded in egg yolk, and frozen coronal sections at 50 µm were obtained through both frontal lobes. Microscopic sections were projected as digitized video images at a final magnification 26 X, and the volume of hematoma in each frontal lobe was calculated using an automated image analysis system (Bioquant System-IV, Nashville, TN). Intracerebral hematomas were classified according to three categories: none (0-10mm³), small (10-50 mm³) or large (>50mm³). Statistical comparison of hemorrhage rates among groups was made using the Pearson chi-square and Fisher's exact test with Yates' correction.

No epidural, subdural, or intraventricular hemorrhages were observed in either saline or heparin treated rats. Subgaleal hematomas were present in 25% of rats receiving heparin and none in the saline-treated group. On gross examination of brains at necropsy, evidence of an intracerebral hematoma correlated well with quantitative measurements from coronal sections.

On histologic section, corticectomy cavities showing no hemorrhage (<10mm³) consisted of Surgicel™ with erythrocytes incorporated in the resorbing cellulose matrix. Small hematomas (10-50 mm³) were composed of distinct collections of intraparenchymal blood filling the corticectomy cavity, but without significant mass effect. Large hematomas (>50 mm³) typically produced a shift of the midline structures and compression of the surrounding brain and ventricular system.

The Table shows the frequency of post-operative hematomas at 10 days after surgery for animals treated for 7 days with saline or heparin. No hemorrhages were observed after placebo or factor XIII application in control (saline-treated) animals. In rats receiving heparin, one small (5%) and 3 large (15%) hematomas were observed in corticectomy cavities treated with topical factor XIII solution, whereas 12 large hematomas (60%) were present in lesions receiving placebo vehicle solution. Compared to placebo treatment, the incidence of intracerebral hemorrhage was significantly lower in corticectomy cavities treated with topical factor XIII (p<0.01).

**TABLE**

| Hematoma Size | Saline | | Heparin | |
|---|---|---|---|---|
| | FXIII | Placebo | FXIII | Placebo |
| None (<10mm³) | 5 | 5 | 16 | 8 |
| Small (10-50mm³) | 0 | 0 | 1 | 0 |
| Large (>50mm³) | 0 | 0 | 3 | 12* |
| Total | 5 | 5 | 20 | 20 |

| | | | | |
|---|---|---|---|---|
| *p < 0.01 | | | | |

## Claims

1. Use of factor XIII for the manufacture of a topically applicable medicament for the inhibition of post-operative haemorrhage at a surgical site in a patient.

2. Use according to claim 1, wherein
(a) the inhibition of post-operative haemorrhage is induction of hemostasis at a surgical site in a patient and
(b) factor XIII is administrable in the form of an effective amount in a biologically compatible vehicle.

3. Use according to claim 2 wherein said patient is coagulopathic.

4. Use according to claim 3 wherein said patient is coagulopathic due to administration of an exogenous anticoagulant or fibrinolytic compound.

5. Use according to claim 2 wherein said surgical site is intracranial.

6. Use according to claim 1 or 2 wherein said factor XIII is recombinant factor XIII.

7. Use according to claim 1 or 2 wherein said factor XIII is a factor XIII **a**₂ dimer.

8. Use according to claim 1 or 2 wherein the concentration of the factor XIII in the vehicle is from 0.1 mg/ml to 100 mg/ml.

9. Use according to claim 1 or 2 wherein the concentration of the factor XIII in the vehicle is from 1.0 mg/ml to 10 mg/ml.

10. Use according to claim 1 or 2 wherein said vehicle is an aqueous diluent.

11. use according to claim 1 or 2 wherein said vehicle is a gel.

12. Use according to claim 2 wherein the step of inducing hemostasis comprises applying a hemostatic sponge, microfibrillar collagen, collagen fleece, or oxidized cellulose to said surgical site.

13. Use according to claim 2 wherein the step of inducing hemostasis comprises cauterizing said surgical site.

## Patentansprüche

1. Verwendung von Faktor XIII zur Herstellung eines topisch applizierbaren Medikaments zur Inhibierung von postoperativer Blutung an einer Operationsstelle bei einem Patienten.

2. Verwendung nach Anspruch 1, worin
(a) die Inhibierung der postoperativen Blutung die Induktion von Hämostase an einer Operationsstelle bei einem Patienten ist und
(b) Faktor XIII in Form einer wirksamen Menge in einem biologisch verträglichen Vehikel verabreichbar ist.

3. Verwendung nach Anspruch 2, worin der Patient koagulopathisch ist.

4. Verwendung nach Anspruch 3, worin der Patient aufgrund der Verabreichung eines exogenen Antikoagulans oder einer fibrinolytischen Verbindung koagulopathisch ist.

5. Verwendung nach Anspruch 2, worin die Operationsstelle in der Schädelhöhle ist.

6. Verwendung nach Anspruch 1 oder 2, worin der Faktor XIII rekombinanter Faktor XIII ist.

7. Verwendung nach Anspruch 1 oder 2, worin der Faktor XIII ein Faktor XIII-**a**₂-Dimer ist.

8. Verwendung nach Anspruch 1 oder 2, worin die Konzentration des Faktors XIII in dem Vehikel 0,1 mg/ml bis 100 mg/ml beträgt.

9. Verwendung nach Anspruch 1 oder 2, worin die Konzentration des Faktors XIII in dem Vehikel 1,0 mg/ml bis 10 mg/ml beträgt.

10. Verwendung nach Anspruch 1 oder 2, worin das Vehikel ein wäßriges Verdünnungsmittel ist.

11. Verwendung nach Anspruch 1 oder 2, worin das Vehikel ein Gel ist.

12. Verwendung nach Anspruch 2, worin der Schritt der Induktion von Hämostase das Aufbringen eines hämostatischen Schwammes, mikrofibrillären Kollagens, eines Kollagen-Vlieses oder von oxidierter Cellulose auf die Operationsstelle umfaßt.

13. Verwendung nach Anspruch 2, worin der Schritt der Induktion von Hämostase die Kauterisation der Operationsstelle umfaßt.

## Revendications

1. Utilisation du facteur XIII pour la fabrication d'un médicament applicable de manière topique afin d'inhiber une hémorragie post-opératoire au niveau d'un site chirurgical chez un patient.

2. Utilisation selon la revendication 1, dans laquelle
(a) l'inhibition d'une hémorragie post-opératoire est une induction d'une hémostase au niveau d'un site chirurgical chez un patient, et
(b) le facteur XIII peut être administré sous la forme d'une quantité efficace dans un véhicule biologiquement compatible.

3. Utilisation selon la revendication 2, dans laquelle ledit patient est coagulopathique.

4. Utilisation selon la revendication 3, dans laquelle ledit patient est coagulopathique du fait d'une administration d'un composé anticoagulant ou fibrinolytique exogène.

5. Utilisation selon la revendication 2, dans laquelle ledit site chirurgical est intracrânien.

6. Utilisation selon la revendication 1 ou 2, dans laquelle ledit facteur XIII est un facteur XIII recombinant.

7. Utilisation selon la revendication 1 ou 2, dans laquelle ledit facteur XIII est un dimère a₂ de facteur XIII.

8. Utilisation selon la revendication 1 ou 2, dans laquelle la concentration du facteur XIII dans le véhicule est comprise entre 0,1 mg/ml et 100 mg/ml.

9. Utilisation selon la revendication 1 ou 2 dans laquelle la concentration du facteur XIII dans le véhicule est comprise entre 1,0 mg/ml et 10 mg/ml.

10. Utilisation selon la revendication 1 ou 2, dans laquelle ledit véhicule est un diluant aqueux.

11. Utilisation selon la revendication 1 ou 2, dans laquelle ledit véhicule est un gel.

12. Utilisation selon la revendication 2, dans laquelle l'étape consistant à induire une hémostase comporte l'application d'un tampon hémostatique, de collagène microfibrillaire, de toile de collagène, ou de cellulose oxydée sur ledit site chirurgical.

13. Utilisation selon la revendication 2, dans laquelle l'étape consistant à induire une hémostase comporte la cautérisation dudit site chirurgical.
